# EUROPEAN PATENT SPECIFICATION

(11) **EP 1 757 601 B1**
(45) Date of publication and mention of the grant of the patent: **05.08.2009**
(21) Application number: 06121714.7
(22) Date of filing: 06.03.2003
(51) Int. Cl.: C07D 401/12, C07D 513/04, A61K 31/473, A61P 27/02

(54) **Quinoline derivatives and their use in glaucoma and myopia**
Chinolinderivate und ihre Anwendung bei Glaukom und Kurzsichtigkeit
Dérivés de quinoline et leur utilisation en glaucome et myopie

(30) Priority: 07.03.2002 EP 02005117
(43) Date of publication of application: 28.02.2007
(62) Divisional of application: 03714784.0
(73) Proprietor: NOVARTIS AG, 4056 Basel (CH); Novartis Pharma GmbH, 1230 Vienna (AT)
(72) Inventor: Gull, Peter, 4148 Pfeffingen (CH); Pombo Villar, Esteban, 4056 Basel (CH)
(74) Representative: von Sprecher, Georg

(56) References cited:
- WO-A-98/01444
- WO-A1-03/074511

## Description

The present invention relates to novel benzo [g] quinoline derivatives, their preparation, their use as pharmaceuticals and pharmaceutical compositions containing them.

More particularly the present invention pertains to a compound of formula 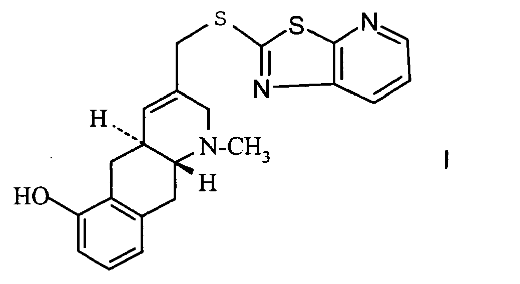 in free base or acid addition salt form.

Acid addition salts may be produced from the free bases in known manner, and vice versa. Suitable acid addition salts for use in accordance with the present invention include for example the hydrochloride.

The compounds of formula I and their physiologically acceptable acid addition salts, referred to hereinafter as compounds of the invention, exhibit valuable pharmacological properties in animal tests and are therefore useful as pharmaceuticals.

### Prior art:

The compounds of the present invention exhibit favorable pharmacological properties vis-à-vis the closest prior art, e.g. EP 912'553.

### Detailed description of the present invention:

In particular, the compounds according to the invention produce a decrease on the intraocular pressure (IOP) in rabbits, at concentrations of 10 to 100 µM. Male rabbits of ca. 2.5 kg are fixed in cages leaving their heads free. The solutions with the compound to be tested are applied to the right eye and the placebo solutions to the left eye (2 drops each, i.e. ca. 40µl). The eyes are firstly anaesthetized with a solution containing Novesine (0.4 %) and Fluorescein (0.05 %) and the ocular pressure is determined at various intervals after administration (10, 20, 30, 60, 90, 120, 180 and 240 minutes), whereby an applanation tonometer according to Goldberg is used.

Surprisingly, the compounds according to the invention exhibit a strong IOP-lowering efficacy, an excellent tolerability, and a long duration of action, and are therefore in particular useful in the treatment of glaucoma and myopia. In a preferred aspect it refers to the treatment of glaucoma.

For the above mentioned indication, the appropriate dosage will of course vary depending upon, for example, the compound employed, the host, the mode of administration and the severity of the condition being treated. However, in general, satisfactory results in animals are indicated to be obtained at a daily dosage of from about 0.1 to about 10 mg/kg animal body weight. In larger mammals, for example humans , an indicated daily dosage is in the range from about 5 to about 200 mg, preferably about 10 to about 100 mg of the compound conveniently administered in divided doses up to 4 times a day or in sustained release form.

The compounds of the invention may be administered in free form or in pharmaceutically acceptable salt form. Such salts may be prepared in conventional manner and exhibit the same order of activity as the free compounds.

Accordingly the present invention provides a compound of the invention for use as a pharmaceutical, e.g. in the treatment of glaucoma and myopia.

The present invention furthermore provides a pharmaceutical composition comprising a compound of the invention in association with at least one pharmaceutically acceptable diluent or carrier. Such compositions may be formulated in conventional manner. Unit dosage forms contain, for example, from about 0.25 to about 50 mg of a compound according to the invention.

Compounds according to the invention may be administered by any conventional route, for example parenterally e.g. in form of injectable solutions or suspensions, or enterally, preferably orally, e.g. in the form of tablets or capsules.

More preferably, they are applied topically to the eye in ca. 0.002 to 0.02 % ophthalmological solutions.

The ophthalmic vehicle is such that the compound is maintained in contact with the ocular surface for a sufficient time period to allow the compound to penetrate the corneal and internal regions of the eye.

The pharmaceutically acceptable ophthalmic vehicle may be e.g. an ointment, vegetable oil, or an encapsulating material.

In accordance with the foregoing, the present invention also provides a compound of the invention for use as a pharmaceutical in the treatment of glaucoma and myopia.

Moreover the present invention provides the use of a compound of the invention, for the manufacture of a medicament for the treatment of glaucoma and myopia.

In still a further aspect the present invention provides a method for the treatment of glaucoma and myopia in a subject in need of such treatment, which comprises administering to such subject a therapeutically effective amount of a compound of the invention.

### Preparative Example 1

To a solution of 4 g (15.4 mM) of 1,2,4aS-trans-5,10,10a-hexahydro-6-methoxy-1-methyl-3-hydroxymethyl-benzo[g]quinoline (see Registry No. 201869-32-1, Chem. Abstr.) in 40 ml of toluene, 1.3 ml (17.9 mM) of thionylchloride are slowly added dropwise at a temperature of 0°C. The suspension is stirred for 12 hours at room temperature. The resulting chloride crystallizes spontaneously. After filtration and washing with toluene, the product is directly used in the next step.

### Preparative Example 2

To a solution of 4 g (17.6 mM) of the chloride as obtained in example 1 in 100 ml dichloromethane, 50.5 ml 1 molar boron tribromide in dichloromethane are slowly added at - 70°C. The suspension is stirred for 3 hours at room temperature, neutralized with NaHCO3 and extracted with ethylacetate / isopropanol (9:1). After drying over Na2SO4, filtering and concentration by evaporation, the corresponding 6-hydroxy-benzo[g]quinoline derivative is directly used in the next step.

### Example 3 => (Compound of formula I)

A solution of 930 mg (3.5 mM) of the 6-hydroxy-benzo[g]quinoline derivative of example 2 and 705 mg (4.2 mM) thiazolo[4,5-b]pyridine-2-thiol in 30 ml dimethylformamide is mixed with 10N NaOH and stirred at room temperature for 1 hour. The resulting suspension is concentrated by evaporation under vacuum and is then extracted with ethylacetate /isopropanol (9:1). After drying over Na₂SO₄, filtration, and removing of the solvent under vacuum, the resulting residue is chromatographed over silica using dichloromethane /ethylacetate / methanol in a ratio of 40:55:5. The purified material is then converted into the hydrochloride by adding HCl in ethanol and subsequent evaporation. Re-crystallization from methanol/ethanol furnished the compound of formula II as hydrochloride, with a m.p. of 240°C (decomp.), α_{D}²⁰= - 101° (c=0.405 in ethanol / water). C₂₁H₂₁N₃OS₂ (HCl), MW = 432.01.

## Claims

1. A compound of formula 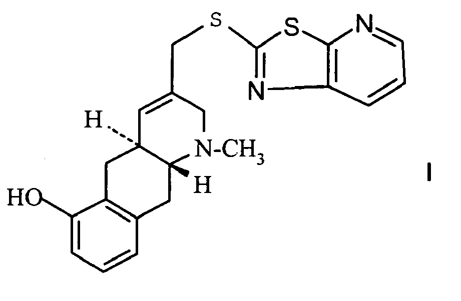 in free base or acid addition salt form.

2. A pharmaceutical composition comprising a compound of claim 1 in free base or pharmaceutically acceptable acid addition salt form and at least one pharmaceutically acceptable diluent or carrier.

3. The compound of claim 1 in free base or pharmaceutically acceptable acid addition salt form for use as a pharmaceutical.

4. The compound of claim 1 in free base or pharmaceutically acceptable acid addition salt form, for use in the treatment of glaucoma and myopia.

5. The use of a compound of claim 1 in free base or pharmaceutically acceptable acid addition salt form, for the manufacture of a medicament for the treatment of glaucoma and myopia.

## Patentansprüche

1. Verbindung der Formel 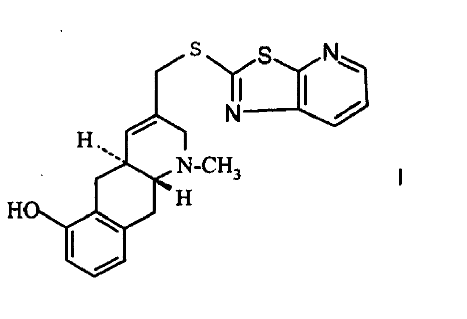 in Form einer freien Base oder in Form eines Säureadditionssalzes.

2. Pharmazeutische Zusammensetzung, umfassend eine Verbindung nach Anspruch 1 in Form einer freien Base oder in Form eines pharmazeutisch akzeptablen Säureadditionssalzes, und wenigstens ein pharmazeutisch akzeptables Verdünnungsmittel oder Träger.

3. Verbindung nach Anspruch 1 in Form einer freien Base oder in Form eines pharmazeutisch akzeptablen Säureadditionssalzes für die Anwendung als ein Pharmazeutikum.

4. Verbindung nach Anspruch 1 in Form einer freien Base oder in Form eines pharmazeutisch akzeptablen Säureadditionssalzes zur Anwendung für die Behandlung von Glaukom und Myopie.

5. Verwendung einer Verbindung nach Anspruch 1 in Form einer freien Base oder in Form eines pharmazeutisch akzeptablen Säureadditionssalzes zur Herstellung eines Arzneimittels für die Behandlung von Glaukom und Myopie.

## Revendications

1. Composé de formule suivante : 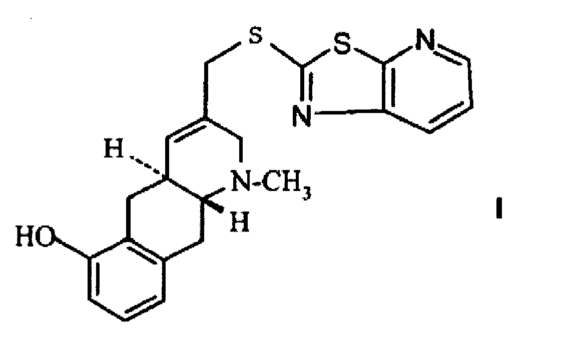 sous forme de base libre ou de sel d'addition d'acide.

2. Composition pharmaceutique comprenant un composé selon la revendication 1 sous forme de base libre ou de sel d'addition d'acide pharmaceutiquement acceptable et au moins un diluant ou support pharmaceutiquement acceptable.

3. Composé selon la revendication 1 sous forme de base libre ou de sel d'addition d'acide pharmaceutiquement acceptable, destiné à être utilisé en tant que produit pharmaceutique.

4. Composé selon la revendication 1 sous forme de base libre ou de sel d'addition d'acide pharmaceutiquement acceptable, destiné à être utilisé dans le traitement du glaucome et de la myopie.

5. Utilisation d'un composé selon la revendication 1 sous forme de base libre ou de sel d'addition d'acide pharmaceutiquement acceptable, pour la fabrication d'un médicament destiné au traitement du glaucome et de la myopie.
